# EUROPÄISCHE PATENTSCHRIFT

(11) **EP 2 271 466 B1**
(45) Veröffentlichungstag und Bekanntmachung des Hinweises auf die Patenterteilung: **04.01.2012**
(21) Anmeldenummer: 09742053.3
(22) Anmeldetag: 05.05.2009
(51) Int. Cl.: B25J 9/16

(54) **MEDIZINISCHER ROBOTER UND VERFAHREN ZUR ERFÜLLUNG DER PERFORMANCEANFORDERUNG EINES MEDIZINISCHEN ROBOTERS**
MEDICAL ROBOT AND METHOD FOR MEETING THE PERFORMANCE REQUIREMENTS OF A MEDICAL ROBOT
ROBOT MÉDICAL ET PROCÉDÉ POUR RÉPONDRE AUX EXIGENCES DE PERFORMANCE D'UN ROBOT MÉDICAL

(30) Priorität: 08.05.2008 DE 102008001664
(43) Veröffentlichungstag der Anmeldung: 12.01.2011
(73) Patentinhaber: KUKA Laboratories GmbH, 86165 Augsburg (DE)
(72) Erfinder: ORTMAIER, Tobias, 30966 Hemmingen (DE); UEBERLE, Marc-Walter, 86316 Friedberg (DE)
(74) Vertreter: Patentanwälte Funk & Böss GbR
(86) Internationale Anmeldenummer: PCT/EP2009/055408
(87) Internationale Veröffentlichungsnummer: WO 2009/135835

(56) Entgegenhaltungen:
- WO-A-98/42482
- WO-A-2005/110267
- WO-A-2008/040426
- WO-A-2008/133956
- DE-U1- 20 010 529
- JP-A- 2002 036 155
- US-A1- 2004 034 282
- US-A1- 2007 144 298

## Beschreibung

Die Erfindung betrifft einen medizinischen Roboter und ein Verfahren zur Erfüllung der Performanceanforderung eines medizinischen Roboters.

Roboter sind Arbeitsmaschinen, die zur automatischen Handhabung und/ oder Bearbeitung von Objekten mit Werkzeugen ausgerüstet werden können und in mehreren Bewegungsachsen beispielsweise hinsichtlich Orientierung, Position und Arbeitsablauf programmierbar sind. Roboter weisen üblicherweise programmierbare Steuerungen (Steuerungsvorrichtungen) auf, die während des Betriebs die Bewegungsabläufe des Roboters steuern.

Roboter können z.B. für medizinische und/oder klinische Anwendungen verwendet werden. Aufgrund eines relativ breiten Anwendungsbereichs in der Medizintechnik ergeben sich deutlich variierende und unter Umständen auch konkurrierende Anforderungen an das geforderte Betriebsverhalten (Performance) des verwendeten Roboters, beispielsweise bezüglich Arbeitsbereich, Steifigkeit, Nutzlast oder Geschwindigkeits- und Beschleunigungskapazität. Die Erfüllung der geforderten Spezifikationen ist gewöhnlich für alle Roboterkonfigurationen zu gewährleisten, insbesondere für den "Worst-Case" der Konfiguration, bei der der entsprechende Performanzkennwert am schlechtesten ist. Dies ist beispielsweise bei dem mechanischen Überlasttest nach IEC 60601-1 gefordert, der im Rahmen der Zulassung von medizintechnischen Geräten z.B. in Deutschland für die ungünstigste Lastkonfiguration im Arbeitsbereich nachgewiesen werden muss. Bei einem gegebenen Arbeitsbereich des Roboters kann dies zu sehr konservativen Leistungsspezifikationen führen.

Die DE 10 2004 043 514 A1 offenbart ein Verfahren und eine Vorrichtung zum Steuern einer sicherheitsrelevanten Funktion einer Maschine. Die Maschine weist eine Maschinensteuerung, einen Sensor zum Erfassen eines Objekts, z.B. einer Person, innerhalb eines Überwachungsbereichs und eine Auswerteeinheit zum Festlegen eines Gefahrenbereichs und zum Auslösen der sicherheitsrelevanten Funktion beim Eindringen des erfassten Objekts in den Gefahrenbereich auf. Zur Festlegung des Gefahrenbereichs ist die Auswerteeinheit mit der Maschinensteuerung gekoppelt und die Auswerteeinheit ist zum Ableiten der für die Festlegung des Gefahrenbereichs erforderlichen Parameter aus den von der Maschinensteuerung zur Bewegungssteuerung der Maschine verwendeten Steuersignalen ausgebildet.

Die Dokumente US 2004/034282 A1 und US 2007/144298 A1 offenbaren Verfahren zur Anpassung des Arbeitsbereichs eines medizinischen Roboters gemäß Performanceanforderungen wie z.B. Geschwindigkeitsbeschränkungen oder Patientbezogenen Sicherheitszonen.

Aufgabe der Erfindung ist es, Voraussetzungen zu schaffen, aufgrund derer die Leistungsspezifikationen des Roboters weniger konservativ gewählt zu werden brauchen.

Die Aufgabe der Erfindung wird gelöst durch ein Verfahren zur Erfüllung der Performanceanforderung eines medizinischen Roboters, aufweisend folgende Verfahrensschritte:
- Befestigen eines medizinischen Werkzeugs an einer Befestigungsvorrichtung eines mehrere Achsen und eine Steuerungsvorrichtung aufweisenden Roboters und
- Anpassen des Arbeitsbereichs des Roboters durch die Steuerungsvorrichtung insbesondere in sicherer Technik derart, dass der Roboter die Performanceanforderung der mit dem medizinischen Werkzeug durchzuführenden Applikation erfüllt.

Die Aufgabe der Erfindung wird auch gelöst durch einen medizinischen Roboter, aufweisend einen Roboterarm mit mehreren bewegbaren Achsen und mit einer Befestigungsvorrichtung und eine Steuerungsvorrichtung zum Bewegen der Achsen des Roboterarms mittels Antrieben, wobei die Steuerungsvorrichtung eingerichtet ist, insbesondere in sicherer Technik den Arbeitsbereich des Roboters derart anzupassen, dass der Roboter eine Performanceanforderung der mit dem medizinischen Werkzeug durchzuführenden Applikation erfüllt.

Mit dem erfindungsgemäßen Roboter kann somit das erfindungsgemäße Verfahren durchgeführt werden.

Ein Aspekt des erfindungsgemäßen Verfahrens ist das Anpassen des Arbeitsbereichs des Roboters, so dass der Roboter die Performanceanforderung der mit dem medizinischen Werkzeug durchzuführenden Applikation erfüllt. Die Performanceanforderung der Applikation umfasst z.B. die geforderte Geschwindigkeitskapazität, die geforderte Beschleunigungskapazität, die auf den Roboter wirkende Last, eine vom Roboter geforderte Steifigkeit aufgrund der Verwendung des Werkzeugs, den Bremsweg des Roboters und/oder eine Manipulierbarkeit des Roboters.

Der Arbeitsbereich eines Roboters ist der zulässige Bereich für den Roboter zum Arbeiten und Verfahren. Im Betrieb des Roboters müssen sich insbesondere alle Achsen des Roboters innerhalb des Arbeitsbereichs befinden. Der Arbeitsbereich ist maximal derjenige Bereich, innerhalb dem sich der Roboter aufgrund seiner mechanischen Ausdehnung bewegen kann. In der Regel ist der Arbeitsbereich jedoch nur ein Teilbereich dieses maximal möglichen Bereichs.

Der Arbeitsbereich eines Roboters kann mechanisch aber auch mittels eines auf der Steuerungsvorrichtung des Roboters laufenden Rechenprogramms begrenzt bzw. eingestellt werden. Nach dem erfindungsgemäßen Verfahren wird der Arbeitsbereich des Roboters von der Steuerungsvorrichtung eingestellt, indem z.B. das besagte Rechenprogramm verhindert, dass im Betrieb des Roboters die Achsen außerhalb des festgesetzten Arbeitsbereichs bewegt werden.

Der erfindungsgemäße Roboter ist insbesondere dafür vorgesehen, mit unterschiedlichen medizinischen Werkzeugen versehen zu werden, d.h. an der Befestigungsvorrichtung des erfindungsgemäßen Roboters sollen im Betrieb insbesondere verschiedene medizinische Werkzeuge, z.B. medizinische Instrumente, befestigt werden. Die Befestigungsvorrichtung ist z.B. ein Flansch des Roboters. Ein solches Szenario ergibt sich insbesondere in der chirurgischen Anwendung, bei der derselbe Roboter für verschiedene Aufgaben eingesetzt wird und potenziell mit verschiedenen Werkzeugen versehen wird. Um den erfindungsgemäßen Roboter nicht für den "Worst-Case" auslegen zu müssen, passt die Steuerungsvorrichtung des erfindungsgemäßen Roboters den Arbeitsbereich an das an der Befestigungsvorrichtung befestigte bzw. zu befestigende Werkzeug an, so dass der Roboter die Performanceanforderung der mit dem medizinischen Werkzeug durchzuführenden Applikation erfüllt. Somit ergibt sich beispielsweise eine dynamische Anpassung des Arbeitsbereichs des Roboters in Abhängigkeit vom verwendeten medizinischen Werkzeug oder auch in Abhängigkeit von dessen Performanceanforderung.

Die Anpassung des Arbeitsbereichs erfolgt z.B. durch eine Beschränkung der Gelenklaufweiten des Roboters, des kartesischen Arbeitsraums und/oder des so genannten Nullraums insbesondere je nach der geforderten Performanceanforderung. Als Nullraum wird der Gelenkwinkelraum eines redundanten Roboters bezeichnet, in dem eine Umkonfiguration der Robotergelenke derart möglich ist, dass die Lage (Position und Orientierung) des Endeffektors des Roboters im Raum unverändert bleibt.

Die Anpassung des Arbeitsbereichs kann insbesondere in sicherer Technik erfolgen.

Eine relativ hohe mechanische Steifigkeit des Roboters kann beispielsweise bei Bohranwendungen in der Orthopädie, bei denen vergleichsweise hohe Interaktionskräfte auftreten können, zur Erreichung einer hohen Eingriffsgenauigkeit notwendig sein. Somit kann der erfindungsgemäße Roboter derart eingerichtet sein, dass dessen Steuerungsvorrichtung bei Verwendung eines für die Bohranwendung vorgesehenen Werkzeugs den Arbeitsbereich auf Regionen relativ hoher Steifigkeit beschränkt, wodurch das für diese Anwendung maßgebliche Performanzkriterium erhöht wird.

Neben der Kraft-/ bzw. Drehmomentkapazität der Aktuatoren des Roboters stellt der z.B. in Deutschland zur Zulassung notwendige Überlasttest nach IEC 60601-1 eine Begrenzung für die Nutzlastkapazität des Roboters dar. Der Roboter muss zur Einhaltung des Überlasttests ein Vielfaches der angegebenen Nutzlast und des Eigengewichts über einen definierten Zeitraum, insbesondere mit aktivierten Bremsen und/oder in Regelung, halten können. Dies ist für alle geplanten Roboterkonfigurationen zu gewährleisten, insbesondere auch für den "Worst Case". Die erfindungsgemäße Anpassung des Arbeitsbereichs des Roboters in Abhängigkeit vom verwendeten medizinischen Werkzeug kann zu einer Erhöhung der zulässigen Nutzlast des Roboters führen.

Anwendungen mit Anforderungen an die zu erreichende Geschwindigkeit des am Roboter befestigten medizinischen Werkzeugs erfordern möglicherweise den Ausschluss von Roboterkonfigurationen in der näheren Umgebung von kinematischen Singularitäten. Eine entsprechende Beschränkung des Arbeitsbereichs kann in diesem Fall zur Einhaltung der geforderten Spezifikation verwendet werden. Anforderungen an die Geschwindigkeitsperformanz finden sich beispielsweise in Anwendungen, bei denen der Roboter die Bewegung von Körperteilen (z.B. Atembewegung) kompensieren soll.

Die Einhaltung der geforderten Beschleunigungskapazität kann möglicherweise nicht im gesamten Arbeitsbereich garantiert und daher nur durch eine Einschränkung des Arbeitsbereiches erreicht werden. Mögliche Applikationen beinhalten wiederum den Einsatz zur Bewegungskompensation. Des Weiteren können Anwendungen mit hohen Sicherheitsanforderungen die Begrenzung des Bremswegs erfordern. Dies kann durch die Beschränkung des Arbeitsbereichs auf Gebiete mit ausreichend hoher Bremsbeschleunigung erreicht werden. Denkbar ist auch eine niedrige Maximalbeschleunigung des Roboters; dies führt im Fehlerfall dazu, dass in der Zeit bis zum Einfallen der Bremsen der Roboter wenig kinetische Energie aufbauen kann und auch wenig Strecke zurücklegt.

Nach einer Variante des erfindungsgemäßen Verfahrens bzw. Roboters ermittelt dieser automatisch Informationen über das am Roboter befestigte Werkzeug, um den Arbeitsbereich zu bestimmen, der die Performanceanforderung erfüllt. Dies kann z.B. dadurch realisiert werden, indem das Werkzeug einen Datenträger aufweist, in dem Daten gespeichert sind, die eine Information über das Werkzeug und/oder über die Performanceanforderung der mit dem medizinischen Werkzeug durchzuführende Applikation aufweisen. Der Datenträger kann beispielsweise mit einem entsprechenden Lesegerät, das z.B. am Roboter angeordnet ist, ausgelesen werden, wodurch es dem erfindungsgemäßen Roboter ermöglicht wird, aufgrund der ausgelesenen Daten automatisch die Steuerungsvorrichtung derart anzupassen, dass diese den Arbeitsbereich des Roboters an das befestigte Werkzeug anpasst. Es kann aber auch vorgesehen sein, dass das Auslesen der Daten manuell initiiert werden muss und/oder dass das Anpassen des Arbeitsbereichs manuell bestätigt werden muss. Die für die Performanceanforderung notwendige Information ist z.B. ein Lastparameter des befestigten Werkzeugs. Ebenso kann die Art der Behandlung eine Rolle bei der Bestimmung der Performanceanforderungen spielen.

Das Auslesen der Daten aus dem Datenträger und/oder das Übertragen der ausgelesenen Daten vom Datenträger zur Steuerungsvorrichtung kann insbesondere in sicherer Technik erfolgen. Die Daten sind insbesondere auch in sicherer Technik gespeichert. Eine sichere Datenübertragung kann z.B. durch die Verwendung einer Checksumme realisiert werden. Es ist auch möglich, dass Daten auf den Datenträger in sicherer Technik zurückgeschrieben werden.

Der Datenträger kann ein kontaktbehafteter oder ein kontaktloser Datenträger sein. Der Datenträger kann insbesondere ein Transponder sein. Ein Transponder ist dabei eine Vorrichtung, die aufgrund eines z.B. von einem Lesegerät stammenden drahtlos empfangenen Signals automatisch ein weiteres Signal erzeugt und drahtlos an das Lesegerät sendet. Transponder bedienen sich der so genannten RFID (Radio Frequncy Identification) Technik und werden auch als RFID-Tags bezeichnet. Transponder können aktive und passive Transponder sein. Aktive Transponder weisen eine eigene aktive Energiequelle, z.B. eine Batterie oder einen Akku, auf. Passive Transponder umfassen dagegen keine aktive Energiequelle und werden durch das elektromagnetische Feld des Lesegerätes mit elektrischer Energie versorgt, indem z.B. ein Kondensator des Transponders durch das elektromagnetische Feld des Lesegerätes geladen wird.

Gemäß einer weiteren Ausführungsform des erfindungsgemäßen Verfahrens bzw. Roboters wird eine Information über das Werkzeug in die Steuerungsvorrichtung z.B. mittels Eingabemittel des Roboters eingegeben, so dass das Anpassen des Arbeitsbereichs an das befestigte oder zu befestigende medizinische Werkzeug aufgrund der eingegebenen Information erfolgen kann.

Da der erfindungsgemäße Roboter im medizinischen oder chirurgischen Umfeld verwendet wird, ist es z.B. möglich, dass für eine bestimmte medizinische Behandlung bzw. für einen bestimmten chirurgischen Eingriff nur ein medizinisches Instrument als das am Roboter zu befestigende Werkzeug verwendet wird. Das Instrument kann z.B. vor dem Eingriff bzw. vor der Behandlung ausgewählt werden. Die Performanceanforderungen können dann von der Applikationssoftware an die Steuerungsvorrichtung insbesondere in sicherer Technik, z.B. über Checksumme gesichert, übermittelt werden. Auf Grundlage dieser Informationen wird der Arbeitsbereich des Roboters, insbesondere ebenfalls in sicherer Technik, beschränkt. Die Beschränkung kann beispielsweise über eine Rechenvorschrift oder über eine zuvor ermittelte Tabelle erfolgen. Ebenso können Geschwindigkeiten, Drehmomente, Beschleunigungen etc. eingeschränkt werden.

Kommen bei dem Eingriff bzw. der Behandlung mehrere Instrumente zum Einsatz und/ oder ändern sich die Performanceanforderungen mit dem Arbeitsschritt, so kann der eben beschriebene Vorgang wiederholt werden.

Wird jedoch ein unbekanntes Instrument verwendet, so kann der Roboter eine automatische Lastermittlung vornehmen, indem das Instrument z.B. in unterschiedliche geeignet gewählte Identifikationsposen gebracht und die zugehörigen Gelenk-Drehmomentwerte ausgelesen werden. Sowohl das Anfahren der Posen als auch das Auslesen der Drehmomente geschieht dabei vorzugsweise in sicherer Technik. Aus dieser Information können nun, bevorzugt ebenfalls in sicherer Technik, die Lastparameter des Instruments berechnet die Performanceanforderungen, insbesondere der Arbeitsbereich beschränkt werden.

Demnach ist es gemäß einer Variante des erfindungsgemäßen Verfahrens bzw. Roboters vorgesehen, wenigstens einen Lastparameter des am Roboter befestigten Werkzeugs durch den Roboter insbesondere in sicherer Technik zu ermitteln, um die Performanceanforderung für das Werkzeugs zu ermitteln. Dies kann durch Bewegen des Werkzeugs mittels des Roboters in unterschiedliche Positionen insbesondere in sicherer Technik und Ermitteln von auf die Achsen des Roboters wirkenden Drehmomenten oder von Antrieben des Roboters aufgebrachten Drehmomenten insbesondere in sicherer Technik erreicht werden. Es ist aber auch möglich, das Werkzeug mittels des Roboters in unterschiedliche Positionen insbesondere in sicherer Technik zu bewegen und von auf die Befestigungsvorrichtung des Roboters wirkenden Kräfte und/oder Drehmomenten insbesondere in sicherer Technik zu ermitteln. Der Lastparameter ist insbesondere die Masse des Werkzeugs und/oder dessen Schwerpunkt.

Ausführungsbeispiele der Erfindung sind exemplarisch in den beigefügten schematischen Zeichnungen dargestellt. Es zeigen:
- Fig. 1: einen Roboter,
- Fig. 2: mehrere Werkzeuge, die am Roboter befestigbar sind,
- Fig. 3: eine Draufsicht des Roboters mit einem Arbeitsbereich des Roboters,
- Fig. 4: eine Seitenansicht des Roboters mit seinem Arbeitsbereich und
- Fig. 5: mehrer Achsenstellungen des Roboters.

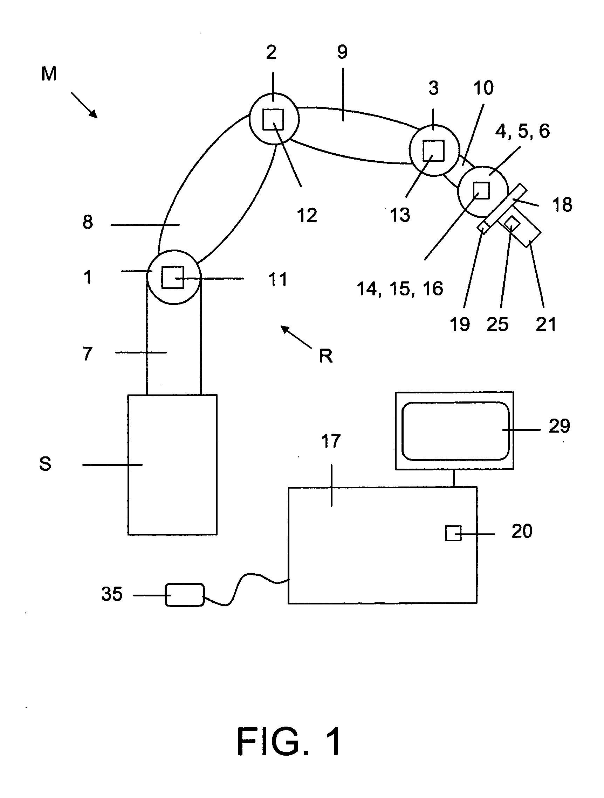

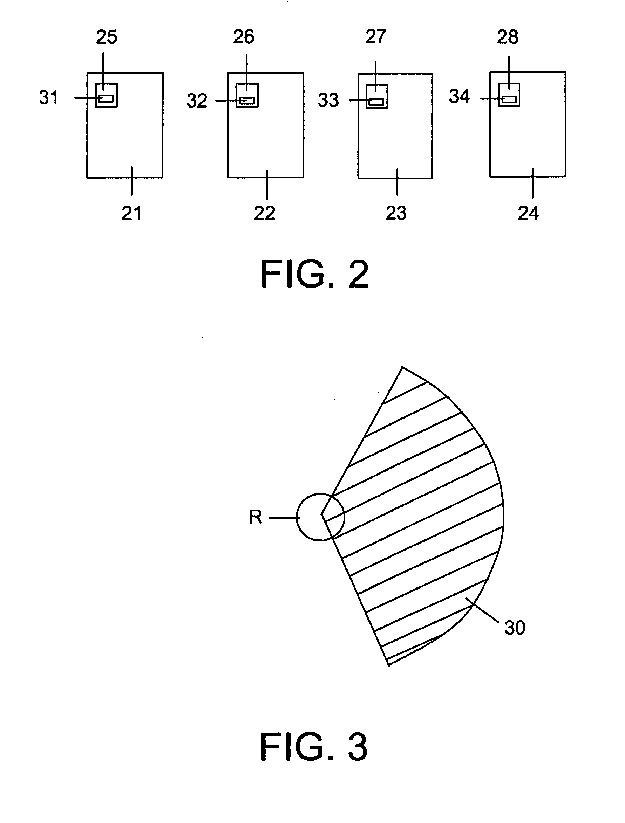

Die Fig. 1 zeigt einen Roboter R mit einem Roboterarm M, der im Falle des vorliegenden Ausführungsbeispiels auf einem Sockel S befestigt ist. Der Roboterarm M stellt im Wesentlichen den beweglichen Teil des Roboters dar und umfasst mehrere Achsen 1-6, mehrere Hebel 7-10 und einen Flansch 18, an dem in der Fig. 2 gezeigte medizinische Werkzeuge 21-24 befestigbar sind. Die Werkzeuge 21-24 können, wie dies im Falle des vorliegenden Ausführungsbeispiels vorgesehen ist, medizinische Instrumente, insbesondere chirurgische Instrumente sein.

Jede der Achsen 1-6 wird mit einem Antrieb, beispielsweise einem elektrischen Antrieb 11-16 bewegt, die in nicht dargestellter Weise mit einem Steuerrechner 17 des Roboters R elektrisch verbunden sind, so dass der Steuerrechner 17 bzw. ein auf dem Steuerrechner 17 laufendes Rechnerprogramm die elektrischen Antriebe 11-16 derart ansteuern kann, dass die Position des Flansches 18 des Roboters im Wesentlichen frei im Raum ausgerichtet werden kann. Die elektrischen Antriebe 11-16 des Roboters R umfassen z.B. jeweils einen elektrischen Motor und gegebenenfalls eine die Motoren ansteuernde Leistungselektronik.

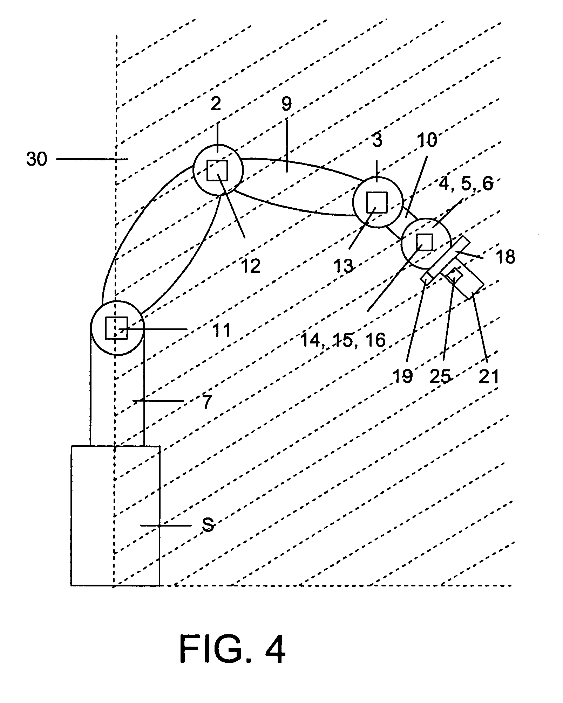

Im Falle des vorliegenden Ausführungsbeispiels ist der Steuerrechner 17 derart ausgeführt, dass er bzw. ein auf ihm laufendes Rechnerprogramm den Arbeitsbereich des Roboters R beschränken kann. Unter einem Arbeitsbereich des Roboters R versteht man den zulässigen Bereich für den Roboter R zum Arbeiten und Verfahren. Im Betrieb des Roboters R müssen sich insbesondere alle Achsen 1-6 des Roboters R innerhalb des Arbeitsbereichs befinden. Die Figuren 3 und 4 zeigen ein Beispiel eines von dem Steuerrechner 17 vorzugsweise in sicherer Technik eingestellten Arbeitsbereichs 30 des Roboters R, wobei die Fig. 3 eine Draufsicht des Roboters R mit Arbeitsbereich 30 und die Fig. 4 eine Seitenansicht des Roboters R mit Arbeitsbereich 30 zeigt.

Im Falle des vorliegenden Ausführungsbeispiels ist es vorgesehen, dass der Steuerrechner 17 den Arbeitsbereich 30 des Roboters R dynamisch an das aktuell am Flansch 18 befestigte Werkzeug 21-24 bzw. an die Performanceanforderung der mit dem aktuell am Flansch 18 befestigten medizinischen Werkzeug 21-24 durchzuführenden Applikation erfüllt.

Damit im Falle des vorliegenden Ausführungsbeispiels der Steuerrechner 17 eine Information erhält, welches der Werkzeuge 21-24 aktuell an seinem Flansch 18 befestigt ist, ist an jedem der Werkzeuge 21-24 jeweils mindestens ein RFID-Transponder 25-28 angeordnet, die im Falle des vorliegenden Ausführungsbeispiels passive Transponder sind. Transponder sind vom Prinzip dem Fachmann bekannt, weshalb die Transponder 25-28 nicht näher dargestellt sind und deren Funktionalität nicht weiter erläutert wird.

Im Falle des vorliegenden Ausführungsbeispiels sind in jedem der Transponder 25-28 Daten 31-34 gespeichert, die eine Information über sein Werkzeug 21-24 umfassen. Diese Information umfasst z.B. die Masse und den Schwerpunkt des relevanten Werkzeugs 21-24. Die Information kann aber auch eine Angabe über die Verwendung des Werkzeugs 21-24 bzw. der Performanceanforderung der entsprechenden medizinischen Applikation umfassen, wie das zugelassene Geschwindigkeitsprofil, das zugelassene Beschleunigungsprofil, oder eine vom Roboter R geforderte Steifigkeit aufgrund der Verwendung des relevanten Werkzeugs 21-24 umfassen.

Ferner ist im Falle des vorliegenden Ausführungsbeispiels am Flansch 18 des Roboters R ein Lesegerät 19 befestigt, das in nicht dargestellter Weise mit dem Steuerrechner 17 verbunden ist.

Wird nun eines der Werkzeuge 21-24 am Flansch 18 des Roboters R befestigt, dann sendet nach dem Beenden des Befestigens das Lesegerät 19 ein Signal aus, das vom Transponder' 25-28 des am Flansch 18 befestigten Werkzeugs 21-24 empfangen wird. In dem in der Fig. 1 dargestellten Ausführungsbeispiel wurde das Werkzeug 21 am Flansch 18 befestigt, woraufhin der Transponder 25 des Werkzeugs 21 das vom Lesegerät 19 erhaltene Signal empfängt und daraufhin automatisch ein Antwortsignal erzeugt und dieses an das Lesegerät 19 sendet. Das Antwortsignal weist die Daten 31 des Werkzeugs 21 auf. Das Lesegerät 19 empfängt das Antwortsignal mit den Daten 31 vom Transponder 25 und übermittelt die Daten 31 an den Steuerrechner 17. Das Auslesen der Daten 31 sowie das Übermitteln der Daten 31 vom Lesegerät 19 zum Steuerrechner 17 erfolgt in sicherer Technik, z.B. unter Verwendung einer Checksumme.

Der Steuerrechner 17 erhält die Daten 31 und ermittelt auf Grundlage der Information über die Performanceanforderung, der Masse und/oder des Schwerpunkts des Werkzeugs 21 den dem Werkzeug 21 zugeordneten Arbeitsbereich 30. Die Ermittlung des Arbeitsbereichs 30 erfolgt z.B. über eine Rechenvorschrift oder über eine zuvor ermittelte und im Steuerrechner 17 abgelegte Tabelle.

Die Ermittlung und Einstellung des dem Werkzeug 21 zugeordneten Arbeitsbereichs 30 kann z.B. automatisch erfolgen. Es ist aber auch möglich, das Auslesen der Daten 31 vom Transponder 25 manuell zu initiieren, indem z.B. eine nicht näher dargestellte Person ein Betätigungsmittel 20 des Steuerrechners 17 betätigt. Es kann zusätzlich oder alternativ vorgesehen sein, die Änderung des Arbeitsbereichs 30 manuell zu bestätigen.

Es ist ebenso möglich über einen sicheren Rückkanal vom Steuerrechner 17 zum Transponder 25 diesen in sicherer Technik zu beschreiben, so dass beispielsweise die Betriebsstunden hinterlegt werden können.

Anstelle einer Speicherung der für das Einstellen des Arbeitsbereichs 30 notwendigen Daten in einem am Werkzeug angeordneten Datenspeicher kann es auch vorgesehen sein, eine Information über das verwendete Werkzeug 21-24 im Steuerrechner 17 zu hinterlegen. Somit ist es möglich, dass die Person am Steuerrechner 17 eine Eingabe über das verwendete Werkzeug 21-24 vornimmt, indem ihr z.B. eine Auswahl von Werkzeugen mittels eines mit dem Steuerrechner 17 verbundenen Bildschirms 29 angeboten wird. Die Person kann dann z.B. durch Anklicken mit einer mit dem Steuerrechner 17 verbundnen Rechnermaus 35 das relevante Werkzeug auswählen. Die Performanceanforderung und die relevanten Lastparameter (Masse und Schwerkraft) des ausgewählten Werkzeugs werden dann von der Applikationssoftware an den Steuerrechner 17 in sicherer Technik, z.B. über Checksumme gesichert, übermittelt. Auf Grundlage dieser Informationen wird der Arbeitsbereich 30 des Roboters R, ebenfalls in sicherer Technik, beschränkt. Ebenso geschieht die Konfiguration durch den Bediener vorzugsweise in sicherer Technik.

Ist keine Information über das Werkzeug 21-24 bzw. dessen Performanceanforderung bekannt, dann ist es im Falle des vorliegenden Ausführungsbeispiels vorgesehen, die Masse und den Schwerpunkt des am Roboter R befestigten Werkzeugs 21-24 folgendermaßen zu ermitteln:

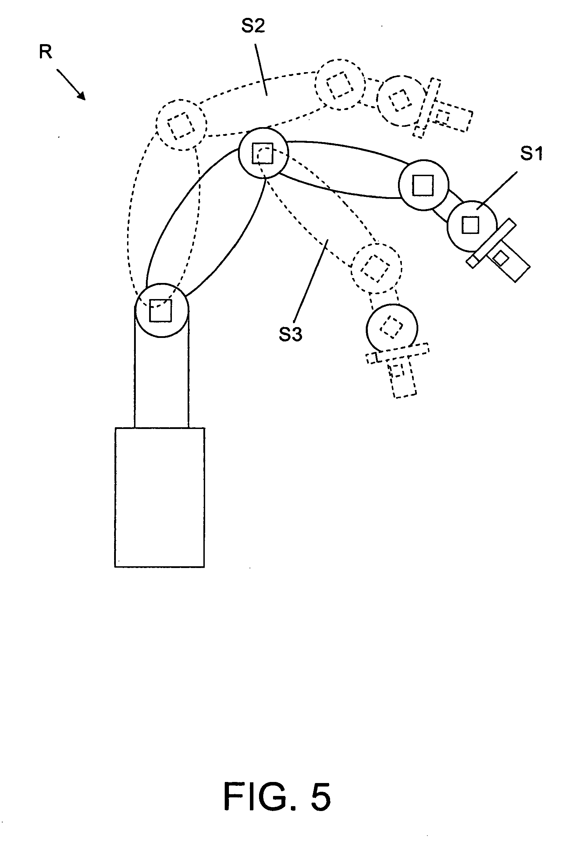

Die Steuervorrichtung 17 steuert die elektrischen Antriebe 11-16 derart an, dass sich die Achsen 1-6 in vorbestimmten Stellungen befinden. In der Fig. 5 ist dies veranschaulicht, indem drei verschiedene Achsstellungen S1-S3 dargestellt sind. Anschließend werden die von den elektrischen Antrieben 11-16 aufgebrachten Drehmomente für die verschiedenen Achsnestellugen S1-S3 z.B. durch Auswerten der elektrischen Ströme der Antriebe 11-16 oder über in den Roboter R integrierte Kraft- und/oder Drehmomentsensoren ermittelt.

Es ist aber auch möglich, dass der Roboter R das an seinem Flansch 18 befestigte Werkzeug 21 in die unterschiedlichen Positionen bewegt und von auf den Flansch 18 wirkenden Kräfte und/oder Drehmomenten ermittelt.

Sowohl das Anfahren der Posen (Achsenstellungen S1-S3) als auch das Bestimmen und/oder die Berechnung der Drehmomente geschieht dabei vorzugsweise in sicherer Technik. Aus dieser Information können nun (ebenfalls in sicherer Technik) die Lastparameter (Masse und Schwerpunkt) des am Roboter R befestigten Werkzeugs berechnet und der relevante Arbeitsbereich 30 entsprechend gesetzt werden.

## Patentansprüche

1. Verfahren zur Erfüllung der Performanceanforderung eines medizinischen Roboters, aufweisend folgende Verfahrensschritte:
- Befestigen eines medizinischen Werkzeugs (21-24) an einer Befestigungsvorrichtung (18) eines mehrere Achsen (1-6) und eine Steuerungsvorrichtung (17) aufweisenden Roboters (R) und
- Anpassen des Arbeitsbereichs (30) des Roboters (R) durch die Steuerungsvorrichtung (17) insbesondere in sicherer Technik derart, dass der Roboter (R) die Performanceanforderung der mit dem medizinischen Werkzeug (21-24) durchzuführenden Applikation erfüllt, **dadurch gekennzeichnet, daß** die Performanceanforderung der Applikation eine vom Roboter (R) geforderte Steifigkeit aufgrund der Verwendung des Werkzeugs (21-24) umfasst.

2. Verfahren nach Anspruch 1, bei dem die Performanceanforderung der Applikation
- die geforderte Geschwindigkeitskapazität,
- die geforderte Beschleunigungskapazität,
- die auf den Roboter (R) wirkende Last,
- der Bremsweg des Roboters (R) und/oder
- eine Manipulierbarkeit des Roboters (R)
umfasst.

3. Verfahren nach Anspruch 1 oder 2, aufweisend automatisches Ermitteln von Informationen über das am Roboter (R) befestigte Werkzeug (21-24), um den Arbeitsbereich (30) zu bestimmen, der die Performanceanforderung erfüllt.

4. Verfahren nach Anspruch 3, aufweisend
- Auslesen von Daten (31-34) aus einem am Werkzeug (21-24) angeordneten Datenträger (25-28), wobei die Daten (31-34) eine Information über das Werkzeug (21-24) und/oder über dessen Performanceanforderung aufweisen, und wobei insbesondere der Datenträger ein Transponder (25-28) ist und die Daten (31-34) mittels eines Lesegeräts (19), das insbesondere am Roboter (R) angeordnet ist, ausgelesen werden, und
- aufgrund der ausgelesenen Daten (31-34), automatisches Anpassen der Steuerungsvorrichtung (17) derart, dass diese den Arbeitsbereich (30) anpasst.

5. Verfahren nach Anspruch 4, zusätzlich aufweisend:
- Manuelles Initiieren des Auslesens der Daten (31-34) nach dem Befestigen des Werkzeugs (21-24) an der Befestigungsvorrichtung (18),
- aufgrund der ausgelesenen Daten (31-34) und aufgrund einer manuellen Bestätigung, Anpassen der Steuerungsvorrichtung (17) derart, dass diese den Arbeitsbereich (30) anpasst, und/oder
- Übertragen der Daten (31-34) vom Datenträger (25-28) zur Steuerungsvorrichtung (17) in sicherer Technik.

6. Verfahren nach Anspruch 1 oder 2, aufweisend Ermitteln von wenigstens einem Lastparameter des am Roboter (R) befestigten Werkzeugs (21-24) durch den Roboter (R) insbesondere in sicherer Technik, um die Performanceanforderung an das Werkzeug (21-24) zu ermitteln.

7. Verfahren nach Anspruch 6, aufweisend, um den Lastparameter zu ermitteln,
- Bewegen des Werkzeugs (21-24) mittels des Roboters (R) in unterschiedliche Positionen (S1-S3) insbesondere in sicherer Technik und Ermitteln von auf die Achsen (1-6) des Roboters (R) wirkenden Drehmomenten oder von Antrieben (11-16) des Roboters (R) aufgebrachten Drehmomenten insbesondere in sicherer Technik oder
- Bewegen des Werkzeugs (21-24) mittels des Roboters (R) in unterschiedliche Positionen (S1-S3) insbesondere in sicherer Technik und Ermitteln von auf die Befestigungsvorrichtung (18) des Roboters (R) wirkenden Kräfte und/oder Drehmomenten insbesondere in sicherer Technik.

8. Verfahren nach einem der Ansprüche 1 bis 7, aufweisend Eingeben einer Information über das Werkzeug (21-24) in die Steuerungsvorrichtung (17) und Anpassen des Arbeitsbereichs (30) aufgrund der eingegebenen Information.

9. Medizinischer Roboter, aufweisend
- einen Roboterarm (M) mit mehreren bewegbaren Achsen (1-6) und mit einer Befestigungsvorrichtung (18) und
- eine Steuerungsvorrichtung (17) zum Bewegen der Achsen (1-6) des Roboterarms (M) mittels Antrieben (11-16), wobei die Steuerungsvorrichtung (17) eingerichtet ist, insbesondere in sicherer Technik den Arbeitsbereich (30) des Roboters (R) derart anzupassen, dass der Roboter (R) eine Performanceanforderung der mit dem medizinischen Werkzeug (21-24) durchzuführenden Applikation erfüllt, **dadurch gekennzeichnet, daß** die Performanceanforderung der Applikation eine vom Roboter (R) geforderte Steifigkeit aufgrund der Verwendung des Werkzeugs (21-24).

10. Roboter nach Anspruch 9, bei dem die Performanceanforderung der Applikation
- die geforderte Geschwindigkeitskapazität,
- die geforderte Beschleunigungskapazität,
- die auf den Roboter (R) wirkende Last,
- der Bremsweg des Roboters (R) und/oder
- eine Manipulierbarkeit des Roboters (R)
umfasst.

11. Roboter nach Anspruch 9 oder 10, der eingerichtet ist, automatisch Informationen über das am Roboter (R) befestigte Werkzeug (21-24) zu ermitteln, um den Arbeitsbereich (30) zu bestimmen, der die Performanceanforderung erfüllt.

12. Roboter nach Anspruch 11, aufweisend eine Lesevorrichtung (19), die eingerichtet ist, Daten (31-34) aus einem an der Befestigungsvorrichtung (18) befestigten Werkzeug (21-24) angeordneten Datenträger (25-28) insbesondere in sicherer Technik auszulesen, wobei die Daten (31-34) eine Information über das Werkzeug (21-24) und/oder über dessen Performanceanforderung aufweisen, und die Steuerungsvorrichtung (17) eingerichtet ist, aufgrund der ausgelesenen Daten (31-34) den Arbeitsbereich (30) anzupassen, wobei insbesondere der Datenträger ein Transponder (25-28), insbesondere ein passiver Transponder ist.

13. Roboter nach Anspruch 9 oder 10, der eingerichtet ist, wenigstens einen Lastparameter eines am Roboter (R) befestigten Werkzeugs (21-24) insbesondere in sicherer Technik zu ermitteln, um die Performanceanforderung zu ermitteln.

14. Roboter nach Anspruch 13, der eingerichtet ist,
- das befestigte Werkzeug (21-24) insbesondere in sicherer Technik in unterschiedliche Positionen (S1-S3) zu bewegen und auf die Achsen (1-6) des Roboters (R) wirkende Drehmomente oder von den Antrieben (11-16) des Roboters (R) aufgebrachte Drehmomente zu ermitteln, um den Lastparameter des befestigten Werkzeugs (21-24) zu ermitteln, oder
- das Werkzeug (21-24) in unterschiedliche Positionen (S1-S3) insbesondere in sicherer Technik zu bewegen und auf die Befestigungsvorrichtung (18) des Roboters (R) wirkenden Kräfte und/oder Drehmomenten insbesondere in sicherer Technik zu ermitteln.

15. Roboter nach einem der Ansprüche 9 bis 14, aufweisend Eingabemittel (35) zum Eingeben einer Information über das Werkzeug (21-24) in die Steuerungsvorrichtung (17), wobei die Steuerungsvorrichtung (17) eingerichtet ist den Arbeitsbereich (30) aufgrund der eingegebenen Information anzupassen.

## Claims

1. Method for complying with the performance requirements of a medical robot, comprising the following method steps:
- securing a medical tool (21-24) to a securing device (18) of a robot (R) which comprises several axes (1-6) and a control device (17) and
- adjusting the operating range (30) of the robot (R) by means of the control device (17), in particular in failsafe technology, such that the robot (R) complies with the performance requirements of the application to be performed by the medical tool (21-24), **characterised in that** the performance requirements of the application include a rigidity required by the robot (R) for the use of the tool (21-24).

2. Method according to claim 1, in which the performance requirements of the application include
- the required speed capacity,
- the required acceleration capacity,
- the load acting on the robot (R),
- the brake path of the robot (R) and/or
- a manipulation ability of the robot (R).

3. Method according to claim 1 or 2, comprising automatically determining information about the tool (21-24) secured to the robot (R), in order to determine the operating range (30) which complies with the performance requirements.

4. Method according to claim 3, comprising
- reading data (31-34) from a data carrier (25-28) arranged on the tool (21-24), wherein the data (31-34) comprises information about the tool (21-24) and/or its performance requirements, and wherein in particular the data carrier is a transponder (25-28) and the data (31-34) is read by means of a reading device (19) which is arranged in particular on the robot (R), and
- on the basis of the read data (31-34) automatically adjusting the control device (17) such that the latter adjusts the operating range (30).

5. Method according to claim 4, also comprising:
- manually initiating the reading of the data (31-34) after securing the tool (21-24) to the securing device (18),
- on the basis of the read data (31-34) and on the basis of manual confirmation, adjusting the control device (17) such that the latter adjusts the operating range (30), and/or
- transmitting the data (31-34) from the data carrier (25-28) to the control device (17) in failsafe technology.

6. Method according to claim 1 or 2, comprising determining at least one load parameter of the tool (21-24) secured onto the robot (R) by the robot (R), in particular in failsafe technology, in order to determine the performance requirements of the tool (21-24).

7. Method according to claim 6, comprising, in order to determine the load parameter,
- moving the tool (21-24) by means of the robot (R) into different positions (S1-S3), in particular in failsafe technology, and determining torques acting on the axes (1-6) of the robot (R) or torques applied by drives (11-16) of the robot (R), in particular in failsafe technology, or
- moving the tool (21-24) by means of the robot (R) into different positions (S1-S3), in particular in failsafe technology, and determining forces and/or torques acting on the securing device (18) of the robot (R), in particular in failsafe technology.

8. Method according to one of claims 1 to 7, comprising entering information about the tool (21-24) into the control device (17) and adjusting the operating range (30) on the basis of the entered information.

9. Medical robot comprising
- a robot arm (M) with several movable axes (1-6) and with a securing device (18) and
- a control device (17) for moving the axes (1-6) of the robot arm (M) by means of drives (11-16), wherein the control device (17) is set up, in particular in failsafe technology, to adjust the operating range (30) of the robot (R) such that the robot (R) complies with performance requirements of the application to be performed by the medical tool (21-24), **characterised in that** the performance requirements of the application include a rigidity required by the robot (R) for the use of the tool (21-24).

10. Robot according to claim 9, in which the performance requirements of the application include
- the required speed capacity,
- the required acceleration capacity,
- the load acting on the robot (R),
- the brake path of the robot (R) and/or
- a manipulation ability of the robot (R).

11. Robot according to claim 9 or 10, which is set up to determine automatically information about the tool (21-24) secured to the robot (R), in order to determine the operating range (30) which complies with the performance requirements.

12. Robot according to claim 11, comprising a reading device (19), which is set up to read data (31-34) from a data carrier (25-28) arranged on the tool (21-24) secured to the securing device (18), in particular in failsafe technology, wherein the data (31-34) includes information about the tool (21-24) and/or its performance requirements, and the control device (17) is set up to adjust the operating range (30) on the basis of the read data (31-34), wherein in particular the data carrier is a transponder (25-28), in particular a passive transponder.

13. Robot according to claim 9 or 10, which is set up to determine at least one load parameter of a tool (21-24) secured to the robot (R), in particular in failsafe technology, in order to determine the performance requirements.

14. Robot according to claim 13, which is set up
- to move the secured tool (21-24) in particular in failsafe technology into different positions (S1-S3) and to determine torques acting on the axes (1-6) of the robot (R) or torques applied by the drives (11-16) of the robot (R), in order to determine the load parameters of the secured tool (21-24), or
- to move the tool (21-24) into different positions (S1-S3) in particular in failsafe technology and to determine forces and/or torques acting on the securing device (18) of the robot (R), in particular in failsafe technology.

15. Robot according to one of claims 9 to 14, comprising input means (35) for entering information about the tool (21-24) into the control device (17), wherein the control device (17) is set up to adjust the operating range (30) on the basis of the entered information.

## Revendications

1. Procédé pour répondre aux exigences de performance d'un robot médical, présentant les étapes de procédé suivantes :
- fixation d'un outil médical (21-24) sur un dispositif de fixation (18) d'un robot (R) présentant plusieurs axes (1-6) et un dispositif de commande (17), et
- adaptation d'une zone de travail (30) du robot (R) par le dispositif de commande (17), en particulier en mode de technique sûre, de telle sorte que le robot (R) répond aux exigences de performance de l'application à exécuter avec l'outil médical (21-24),
- **caractérisé en ce que** les exigences de performance de l'application comprennent une raideur requise par le robot (R) en raison de l'utilisation de l'outil (21-24).

2. Procédé selon la revendication 1, dans lequel les exigences de performance comprennent
- la capacité de vitesse requise,
- la capacité d'accélération requise,
- la charge agissant sur le robot (R),
- la distance de freinage du robot (R) et/ou
- une manipulabilité du robot (R).

3. Procédé selon les revendications 1 ou 2, présentant la détermination automatique d'informations concernant l'outil (21-24) fixé sur le robot (R) pour déterminer la zone de travail (30) qui répond aux exigences de performance.

4. Procédé selon la revendication 3, présentant
- la lecture de données (31-34) depuis un support de données (25-28) agencé sur l'outil (21-24), les données (31-34) présentant une information sur l'outil (21-24) et/ou sur ses exigences de performance, et en particulier le support de données (31-34) étant un transpondeur (25-28), et les données (31-34) étant lues au moyen d'un lecteur (19) qui est en particulier agencé sur le robot (R), et
- sur la base des données (31-34) lues, l'adaptation du dispositif de commande (17), de telle sorte que celui-ci adapte la zone de travail (30).

5. Procédé selon la revendication 4, présentant en outre :
- l'initiation manuelle de la lecture des données (31-34) après avoir fixé l'outil (21-24) sur le dispositif de fixation (18),
- sur la base des données (31-34) lues et sur la base d'une confirmation manuelle, adaptation du dispositif de commande (17) de telle sorte que celui-ci adapte la zone de travail (30), et/ou
- transmission des données (31-34) depuis le support de données (25-28) au dispositif de commande (17) en mode de technique sûre.

6. Procédé selon les revendications 1 ou 2, présentant la détermination, par le robot (R), d'au moins un paramètre de charge de l'outil (21-24) fixé sur le robot (R), en particulier en mode de technique sûre, pour déterminer les exigences de performance requises pour l'outil (21-24).

7. Procédé selon la revendication 6, présentant, pour déterminer le paramètre de charge,
- le déplacement de l'outil (21-24) au moyen du robot (R) dans différentes positions (S1-S3), en particulier en mode de technique sûre, et détermination de couples de rotation agissant sur les axes (1-6) du robot (R) ou de couples de rotation introduits par des entraînements (11-16) du robot (R) en mode de technique sûre, ou
- le déplacement de l'outil (21-24) au moyen du robot (R) dans différentes positions (S1-S3) en particulier en mode de technique sûre, et détermination de forces et/ou de couples de rotation agissant sur le dispositif de fixation (18) du robot (R), en particulier en mode de technique sûre.

8. Procédé selon l'une des revendications 1 à 7, présentant l'introduction d'une information via l'outil (21-24) dans le dispositif de commande (17) et adaptation de la zone de travail (30) sur la base de l'information introduite.

9. Robot médical, présentant
- un bras de robot (M) avec plusieurs axes mobiles (1-6) et avec un dispositif de fixation (18), et
- un dispositif de commande (17) pour déplacer les axes (16) du bras de robot (M) au moyen d'entraînements (11-16), le dispositif de commande (17) étant aménagé de manière à adapter, en particulier en mode de technique de sécurité, la zone de travail (30) du robot (R) de telle sorte que le robot (R) réponde à des exigences de performance de l'application à exécuter avec l'outil (21-24) médical, **caractérisé en ce que** les exigences de performance de l'application est une raideur requise par le robot (R) en raison de l'utilisation de l'outil (21-24).

10. Robot selon la revendication 9, dans lequel les exigences de performance de l'application comprennent
- la capacité de vitesse requise,
- la capacité d'accélération requise,
- la charge agissant sur le robot (R),
- la distance de freinage du robot (R) et/ou
- une manipulabilité du robot (R).

11. Robot selon les revendications 9 ou 10. qui est aménagé de manière à déterminer automatiquement des informations concernant l'outil (21-24) fixé sur le robot (R) pour déterminer la zone de travail (30) qui répond aux exigences de performance.

12. Robot selon la revendication 11, présentant un dispositif de lecture (19) qui est aménagé de manière à lire des données (31-34) depuis un support de données (31-34) agencé sur un outil (21-24) fixé sur le dispositif de fixation (18), en particulier en mode de technique sûre, les données (31-34) présentant une information sur l'outil (21-24) et/ou sur ses exigences de performance, et le dispositif de commande (17) étant aménagé de manière à adapter la zone de travail (30) sur la base des données (31-34) lues, le support de données étant en particulier un transpondeur (25-28), en particulier un transpondeur passif.

13. Robot selon les revendications 9 ou 10, qui est aménagé de manière à déterminer au moins un paramètre de charge d'un outil (21-24) fixé sur le robot (R), en particulier en mode de technique sûre, pour déterminer les exigences de performance.

14. Robot selon la revendication 13, qui est aménagé de manière à :
- déplacer l'outil (21-24) fixé dans différentes positions (S1-S3), en particulier en mode de technique sûre, et déterminer des couples de rotation agissant sur les axes (1-6) du robot (R) ou des couples de rotation introduits par les entraînement (11-16) du robot (R) pour déterminer le paramètre de charge de l'outil (21-24) fixé,
- déplacer l'outil (21-24) dans différentes positions (S1-S3), en particulier en mode de technique sûre, et déterminer des forces et/ou couples de rotation agissant sur le dispositif de fixation (18) du robot (R), en particulier en mode de technique sûre.

15. Robot selon l'une des revendications 9 à 14, présentant des moyens d'introduction (35) pour introduire une information concernant l'outil (21-24) dans le dispositif de commande (17), le dispositif de commande (17) étant aménagé de manière à adapter la zone de travail (30) sur la base de l'information introduite.
